Europäisches Patentamt

European Patent Office

Office européen des brevets

(19)

(11) Publication number: **0 076 870**
**B1**

(12) **EUROPEAN PATENT SPECIFICATION**

(45) Date of publication of patent specification: **05.06.85**

(21) Application number: **81108210.6**

(22) Date of filing: **12.10.81**

(51) Int. Cl.⁴: **A 61 K 35/16,** C 07 K 3/12,
C 07 K 15/06

(54) Process for the preparation of highly purified antihemophilic factor.

(43) Date of publication of application:
**20.04.83 Bulletin 83/16**

(45) Publication of the grant of the patent:
**05.06.85 Bulletin 85/23**

(84) Designated Contracting States:
**AT BE CH DE FR GB IT LI LU NL SE**

(56) References cited:
**EP-A-0 033 582**
**EP-A-0 035 202**
**FR-A-2 293 943**
**FR-A-2 473 886**
**US-A-3 973 002**
**US-A-4 104 266**
**US-A-4 170 639**

**HAEMOSTASIS, vol.10, Supplement 1, 1981, F.
FELDMANN: "Characterization of a new
commercial AHF preparation of high purity:
Factorate generation II", page 205
BIOLOGICAL ABSTRACTS, vol.60, May 1975,
abstract no.64720, C.K. KASPER et al.:
"Determinants of factor VIII recovery in
cryoprecipitate"**

(73) Proprietor: **Armour Pharmaceutical Company
303 South Broadway
Tarrytown New York 10591 (US)**

(72) Inventor: **Feldmann, Fred
407 Gettysburg Street
Park Forest Illinois (US)**

(74) Representative: **Hansen, Bernd, Dr.rer.nat. et al
Hoffmann, Eitle & Partner Patentanwälte
Arabellastrasse 4
D-8000 München 81 (DE)**

(56) References cited:
**CHEMICAL ABSTRACTS, vol.82, no.17, April 28,
1975, page 209, abstract no.108318s,
Columbus, Ohio (US), M.E. LEGAZ et al.:
"Isolation, subunit structure, and proteolytic
modification of bovine factor VIII"**

Courier Press, Leamington Spa, England.

## Description

This invention relates to antihemophilic factor present in human blood plasma. It particularly relates to compositions containing antihemophilic factor (AHF) of high potency and purity and substantially free of denatured AHF and to processes for the preparation thereof.

The isolation of AHF by fractionation from other proteins present in blood plasma has been achieved. The prior art of AHF fractionation has demonstrated that AHF can be separated in part or in toto from fibrinogen and other proteins by column chromatography, polyethylene glycol (PEG) or polypropylene glycol (PPG) precipitation, glycine precipitation (or with other amino acids), as well as alcohol precipitation. British patent no. 1,507,198 and U.S. patent no. 3,973,002 utilize pH and temperature adjustments to result in some purification of AHF, but the procedure described in these patents uses a starting material different from what is used in the process described in this application, and results in low potency preparations of intermediate purity. The processes described in these patents utilize a fraction extracted from the cryoprecipitate rather than cryoprecipitate itself. Furthermore, fractionation stops after one cycle of pH and cooling adjustment. Others have also recognized the effect of using one cycle of pH and cooling adjustment (J. K. Smith et al., *Transfusion 19*, 299—306, (1979)).

US—A—4 170 639 is concerned with an antihemophilic factor (AHF) concentrate having enhanced potency and solubility and its process of production. The process includes the steps of removing unwanted protein from an aqueous extract of antihemophilic blood plasma cryoprecipitate by mixing the aqueous extract with aluminium hydroxide at an acid pH in the cold, adjusting the purified aqueous extract to an acid pH and freeze-drying the extract, optionally removing water from the extract just before freeze-drying.

The compositions of AHF obtained by the prior art processes discussed above are of relatively low concentration (of the order of about 5 to 15 units of AHF activity per ml) and low purity (less than 1 unit AHF activity per mg protein), one of the undesirable impurities being denatured AHF.

The present invention provides an improved process for preparing from aqueous compositions containing AHF obtained from human plasma, AHF compositions of high potency and purity and substantially free of denatured AHF.

The present invention provides an aqueous composition of high concentration (about 20 to 45 units of AHF activity per ml) and high potency (at least 1 unit AHF activity per mg of protein) and dried compositions having 1 to 10 units AHF per mg protein, such compositions being substantially free of denatured AHF, from human plasma by the process described below.

The process of the present invention may be applied to any aqueous composition of AHF having about 5 to 15 units AHF activity per ml, and a specific activity of about 0.3 to 1.0 unit AHF activity per mg of protein.

In accordance with the present invention such aqueous composition is subjected to the following steps:

(a) effecting about a 2- to 10-fold concentration of the solution,

(b) adjusting the pH of said concentrated solution to about 5.0 to 6.0,

(c) cooling resultant solution to about 0 to 14°C,

(d) removing the precipitate which forms at said pH and temperature,

(e) further clarifying the solution by filtration or other equivalent means, and

(f) adjusting the pH to about 7.0.

The resulting solution is subjected to sterile filtration and the filtrate obtained therefrom may be put directly into sterile vials or freeze-dried to form a sterile dry preparation. The solutions and dry preparations may be marketed directly as such.

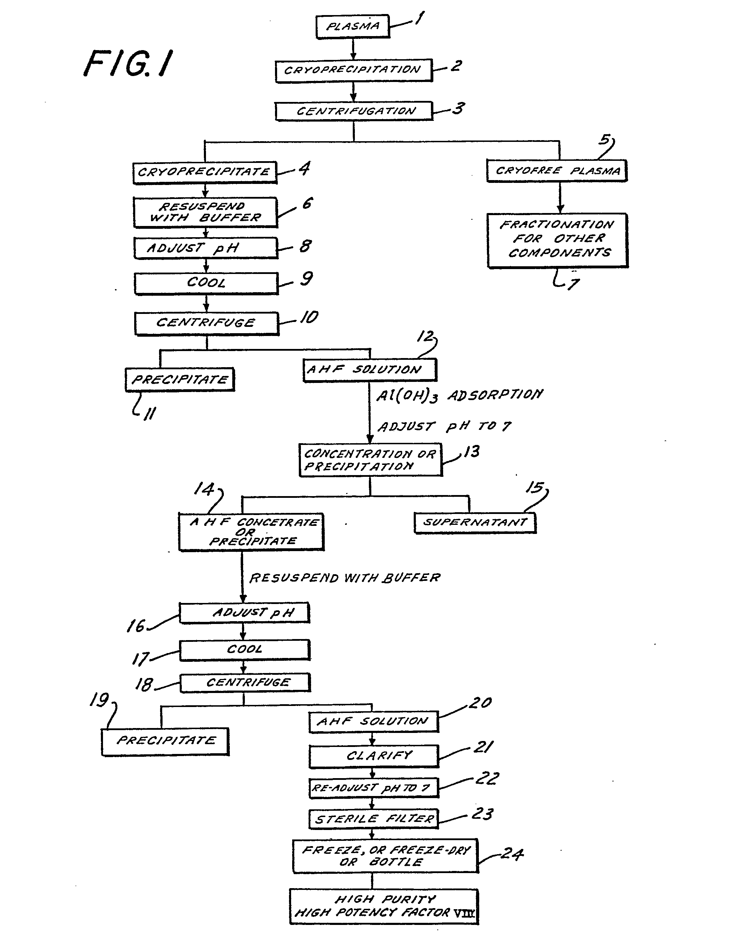

The present invention will be illustrated in the description which follows, with reference to Figure 1 which is a flow sheet illustrating steps of a prior art process and the steps of the process of the present invention. This description is given by way of illustration and is not to be considered as limiting.

Human plasma is subjected to freezing, thawing, and centrifugation resulting in the removal of the cryoprecipitate. At physiological pH and temperature (pH 7—7.4, room temperature to 37°C), the cryoprecipitate (#4) can be dissolved in a buffer (such as tris hydrochloride or glycine with saline and sodium citrate) to give a uniform solution. The cryoprecipitate, or a concentrated fraction from the cryoprecipitate contains a complex mixture of clotting proteins (including AHF), fibrinogen, albumin, and other proteins. By acidifying the resuspended cryoprecipitate (#6) (to a pH 6.0—6.8) (step #8) and cooling (to 5—15°C) (step #9) some of the proteins (such as the fibrinogen and the cold-insoluble globulins can be made to precipitate (step #10). By carefully choosing the right conditions, as described above (pH 6—6.8, temperature 5—15°C), AHF can be made to remain in the supernatant (step #12).

If the pH is made more acidic than pH 6.4 and the solution cooled at this step, (steps 8—12), more proteins are precipitated and can be removed from the solution, but AHF is also precipitated resulting in no additional purification. The processes described in the prior art do not go beyond this stage, and this had led to the assumption that further purification is not possible by the continued manipulation of pH and temperature.

In accordance with the present invention a high purity concentrate of AHF is obtained by the additional steps described below, reference, as noted above, being made to the flow sheet in Figure 1.

The separated AHF solution (#12) after Al(OH)$_3$ absorbtion is concentrated or precipitated. The concentrated AHF solution (#14) as high potency (25—45 units per ml), but intermediate purity (less than one AHF unit per mg protein). The concentrated AHF solution (#14) (or concentrated resuspended AHF solution (#14) is then adjusted to pH 5.0—6.0 and cooled to 0—14°C at which temperature a precipitate forms. The precipitate (#19) is removed and the AHF (#20) solution clarified, adjusted to pH 7, sterile filtered and bottled or bottled and freeze-dried.

The sterile solution contains about 25 to 45 units per ml, has a potency of about 1 to 10 units AHF activity per mg of protein and is substantially free of denatured AHF.

The freeze-dried preparation contains about 1 to 10 units AHF activity per mg protein and is also substantially free of denatured AHF.

The present invention teaches, therefore, that if the AHF solution from the acidified and cooled steps (steps 8—12) is readjusted to pH 7 and concentrated, that subsequent acidification and cooling effect the removal by precipitation of additional large amounts of fibrinogen, plasminogen and plasmin, cold insoluble globulins, and other proteins without coprecipitation of AHF, and result in a gentle conversion from an AHF preparation of intermediate potency and purity to a preparation of high potency and purity.

Attempting to extend the first acidification and cooling (steps 8—12) without this sequence is fruitless.

An additional benefit of this purification is that it is a gentle procedure and results in an AHF preparation which is native, that is, one in which the ratio of antigenic Factor VIII (FVIII Coagulant Antigen) to Factor VIII procoagulant activity is approximately one, indicating the absence of denatured AHF (one which shows substantial levels of inactive antigenic Factor VIII). No other purification scheme has been demonstrated to produce this unique result.

## Claims

1. A process for the preparation of an aqueous composition of antihemophilic factor, having a concentraltion of 20 to 45 units of antihemophilic factor per ml, a specific antihemophilic factor activity of at least 1 unit per mg protein, and being obtained from an aqueous solution of antihemophilic factor derived from human blood plasma, said aqueous solution having a concentration of 5 to 15 units of antihemophilic factor per ml, and a specific antihemophilic factor activity of 0.3 to 1.0 unit per mg protein, which comprises:

(a) effecting a 2- to 10-fold concentration of the low concentrate solution,

(b) adjusting the pH of said concentrated solution to 5.0 to 6.0,

(c) cooling the resultant solution to 0 to 14°C,

(d) removing the precipitate which forms at said pH and temperature,

(e) further clarifying the solution from step (d), and

(f) adjusting the pH to about 7.0.

2. Use of an aqueous solution of antihemophilic factor, having a concentration of 5 to 15 units of antihemophilic factor per ml, said antihemophilic factor having a specific antihemophilic factor activity of 0.3 to 1 unit per mg protein, obtained from blood plasma by

(a) cryoprecipitating the antihemophilic factor,

(b) dissolving the cryoprecipitating in a buffer at pH 7.0 to 7.4,

(c) precipitating fibrinogen and cold insoluble globulen by adjusting the pH to 6.0 to 6.8 and the temperature to 5 to 15°C and removing the precipitate,

(d) removing the prothombin complex in the resulting solution by adsorbing on aluminum hydroxide, and

(e) recovering the resulting solution, as starting solution in a process according to claim 1.

## Revendications

1. Procédé de préparation d'une composition aqueuse de facteur anti-hémophilique ayant une concentration de 20 à 45 unités de facteur anti-hémophilique par ml, une activité spécifique de facteur anti-hémophilique d'au moins 1 unité par mg de protéine, et que l'on tire d'une solution aqueuse de facteur anti-hémophilique tiré du plasma sanguin humain, cette solution aqueuse ayant une concentration de 5 à 15 unités de facteur anti-hémophilique par ml et une activité spécifique de facteur anti-hémophilique de 0,3 à 1,0 unité par mg de protéine, caractérisé en ce qu'il consiste:

(a) à effectuer une concentration, au coefficient 2 à 10, de la solution peu concentrée,

(b) à régler le pH de la solution concentrée à 5,0 à 6,0,

(c) à refroidir la solution obtenue à 0 à 14°C,

(d) à éliminer le précipité qui se forme à ce pH et à cette température,

(e) à clarifier davantage la solution provenant de l'étape (d), et

(f) à régler le pH à environ 7,0.

2. Utilisation d'une solution aqueuse de facteur anti-hémophilique ayant une concentration de 5 à 15 unités de facteur anti-hémophilique par ml, ce facteur anti-hémophilique ayant une activité spécifique de facteur hémophilique de 0,3 à 1 unité par mg de protéine, tirée du plasma sanguin par

(a) cryoprécipitation du facteur anti-hémophilique,

(b) dissolution du cryoprécipité dans un tampon à pH 7,0 à 7,4,

(c) précipitation du fibrinogène et des globulines insolubles à froid par réglage du pH à 6,0 à 6,8 et de la température à 5 à 15°C et élimination du précipité,

(d) élimination du complexe de prothrombine dans la solution obtenue, par adsorption sur de l'hydroxyde d'aluminium, et

5

0 076 870

6

(e) récupération de la solution obtenue, en tant que solution de départ dans un procédé selon la revendication 1.

**Patentansprüche**

1. Verfahren zur Herstellung einer wässrigen Zusammensetzung des antihämophilen Faktors mit einer Konzentration von 20 bis 45 Einheiten des antihämophilen Faktors pro ml, einer spezifischen antihämophilen Faktoraktivität von wenigstens 1 Einheit pro mg Protein, die erhalten wurde aus einer wässrigen Lösung aus antihämophilen Faktors, erhalten aus Human-Blutplasma, wobei die wässrige Lösung eine Konzentration von 5 bis 15 Einheiten an antihämophilem Faktor pro ml und eine spezifische antihämophile Faktoraktivität von 0,3 bis 1,0 Einheiten pro mg Protein aufweist, dadurch gekennzeichnet, dass man

(a) eine 2- bis 10-fache Konzentration der niedrigkonzentrierten Lösung vornimmt,

(b) den pH der konzentrierten Lösung auf 5,0 bis 6,0 einstellt,

(c) die erhaltene Lösung auf 0 bis 14°C kühlt,

(d) den Niederschlag, der sich bei dem genannten pH-Wert und der genannten Temperatur bildet, entfernt,

(e) die Lösung aus Stufe (d) weiter klärt und

(f) den pH auf etwa 7,0 einstellt.

2. Verwendung einer wässrigen Lösung eines antihämophilen Faktors mit einer Konzentration von 5 bis 15 Einheiten an antihämophilem Faktor pro ml, wobei der antihämophile Faktor eine spezifische antihämophile Faktoraktivität von 0,3 bis 1 Einheit pro mg Protein aufweist und das Blutplasma erhalten wurde durch

(a) Ausfällen des antihämophilen Faktors bei niedrigen Temperaturen,

(b) Auflösen des Cryoprecipitats in einer Pufferlösung bei pH 7,0 bis 7,4,

(c) Ausfällen von Fibrinogen und in der Kälte unlöslichem Globulen durch Einstellen des pH's auf 6,0 bis 6,8 und der Temperatur auf 5 bis 15°C und Entfernen des Niederschlags,

(d) Entfernen des Prothrombin-Komplexes in der erhaltenen Lösung durch Adsorption auf Aluminiumhydroxid, und

(e) Gewinnen der erhaltenen Lösung als Ausgangslösung in einem Verfahren gemäss Anspruch 1.

0 076 870

*FIG.1*